# EUROPEAN PATENT SPECIFICATION

(11) **EP 3 583 939 B1**
(45) Date of publication and mention of the grant of the patent: **09.08.2023**
(21) Application number: 19382505.6
(22) Date of filing: 18.06.2019
(51) Int. Cl.: A61K 9/51, A61K 31/573, C07C 69/54, C08F 226/10, C08J 3/14

(54) **AUTOASSOCIATIVE ACRYLIC FUNCTIONAL COPOLYMERS AND TERPOLYMERS AND THEIR USE AS VEHICLES OF BIOACTIVE COMPOUNDS**
AUTOASSOZIATIVE ACRYLFUNKTIONELLE COPOLYMERISATE UND TERPOLYMERE UND IHRE VERWENDUNG ALS TRÄGER VON BIOAKTIVEN VERBINDUNGEN
COPOLYMÈRES ET TERPOLYMÈRES ACRYLIQUES AUTOASSOCIATIFS ET LEURS UTILISATIONS COMME TRANSPORTEURS DE COMPOSES BIOACTIFS

(30) Priority: 18.06.2018 ES 201830594
(43) Date of publication of application: 25.12.2019
(73) Proprietor: Alodia Farmacéutica, S.L., 28760 Tres Cantos (Madrid) (ES); Centro de Investigación Biomedica en Red en Bioingenieria, Biomateriales y Nanomedicina, 50018 Zaragoza (ES); Consejo Superior de Investigaciones Cientificas, 28006 Madrid (ES)
(72) Inventor: SAN ROMÁN DEL BARRIO, Julio, 28006 Madrid (ES); AGUILAR DE ARMAS, María Rosa, 28006 Madrid (ES); PALAO SUAY, Raquel, 28006 Madrid (ES); NAKAL, Alberto, 28006 Madrid (ES)
(74) Representative: Pons Ariño, Angel

(56) References cited:
- FR-A1- 2 952 936
- RAQUEL PALAO-SUAY ET AL: "Anticancer and Antiangiogenic Activity of Surfactant-Free Nanoparticles Based on Self-Assembled Polymeric Derivatives of Vitamin E: Structure-Activity Relationship", BIOMACROMOLECULES, vol. 16, no. 5, 22 April 2015 (2015-04-22) , pages 1566-1581, XP055632704, US ISSN: 1525-7797, DOI: 10.1021/acs.biomac.5b00130
- ORTIZ C ET AL: "Synthesis, characterization and properties of polyacrylic systems derived from vitamin E", POLYMER, ELSEVIER SCIENCE PUBLISHERS B.V, GB, vol. 39, no. 17, 1 August 1998 (1998-08-01), pages 4107-4114, XP004120962, ISSN: 0032-3861, DOI: 10.1016/S0032-3861(97)10375-5

## Description

The present invention relates to a family of amphiphilic terpolymers forming nanometric-sized bioactive polymeric multi-micellar nanoparticles, consisting of synthetic polymer systems derived from acrylic functional monomers containing α-tocopherol (vitamin E) as a bioactive and hydrophobic component, and hydrophilic monomers consisting of N-vinylpyrrolidone and N-vinyl caprolactam. These compounds can be used as controlled release systems for the administration of bioactive compounds.

### BACKGROUND OF THE INVENTION

The breakthroughs made in nanotechnologies in recent years have allowed the development of very specific systems for highly targeted applications in the area of regenerative medicine, offering new biomedical treatment strategies providing very novel and interesting solutions to problems that are difficult to predict and effectively treat, such as intraarticular applications to improve joint biomechanics, as well as to procure an effective treatment of osseo-articular regeneration, or epidermal treatments to improve conditions of the epidermis.

〈-Tocopherol (vitamin E) is highly hydrophobic, exhibits very low or zero solubility in physiological media and, therefore, is difficult to administer and maintain in the physiological medium in an active form. Acrylic derivatives of vitamin E (either acrylate or methacrylate) exhibit the same hydrophobic character, but as a result of their reactivity due to the acrylic component, they can be used for preparing polymer systems in a wide range of compositions by polymerization with relevant vinyl derivatives, among which N-vinyl pyrrolidone and N-vinyl caprolactam are considered. The result is a biocompatible polymer system with a high molecular weight that is notably amphiphilic, depending on the content of hydrophilic and hydrophobic components. As a consequence, the system acts like a macromolecular compound, with a controlled molecular weight and being distinctly amphiphilic, which is autoassociative under physiological conditions to form nanoparticles with a size controlled depending on the methodology applied in its preparation. The bioactive component (vitamin E) is therefore chemically anchored to a micellar system which can offer, on one hand, self-assembly capacity, and at the same time offer very considerable antioxidant activity at the cell level. Depending on the composition and the molecular weight, it is possible to prepare stable self-assembled nanoparticles under physiological conditions with variable sizes that can range between 50 nm and 500 nm. These nanoparticles, which are stable in aqueous media and physiological medium, can be effectively dispersed in polymer solutions in the form of a gel or high viscosity solutions with resorbable and biodegradable polymers such as chitosan, hyaluronic acid, chondroitin sulfate, gelatin, acacia gum, Aloe Vera, and cellulose derivatives such as methyl cellulose, carboxymethyl cellulose, starches, dextrins, etc.

The scientific paper entitled 'Anticancer and Antiangiogenic Activity of Surfactant-Free Nanoparticles Based on Self-Assembled Polymeric Derivatives of Vitamin E: Structure-Activity Relationship" R. Palao-Suay et al. Biomacromolecules (2015) 16 1566-1581, describes acrylic polymeric derivatives carrying 〈-tocopherol in up to 20 % of the total monomers in the feed, where 〈-tocopherol is directly attached to the acrylic group of the polymeric derivative. The application of these systems is oriented in relation to the antioxidant capacity associated with the 〈-tocopherol that is included.

The patent FR2 952 936 A1 describes copolymers and terpolymers of acrylic or methacrylic monomers that include an α-tocopherol-containing (meth)acrylic monomer and two further hydrophilic monomers, that can form nanoparticles in aqueous media at neutral pH.

Therefore, from a viewpoint applied to developing systems, it would be of enormous interest for them to offer self-assembly capacity, to simultaneously be carriers of the antioxidant component par excellence (vitamin E), and to enable being used in the form of nanoparticles as vehicles for vectorization and controlled and targeted release of other bioactive components which can be loaded into or added to the nanoparticles in suitable proportions, notably decreasing their toxicity and allowing their application to a certain point of the body (targeted and localized application), whereby substantially reducing their toxic side effects and achieving an efficacy superior to that which would be obtained by the application of bioactive components applied in a free or isolated manner.

### DESCRIPTION OF THE INVENTION

The present invention describes the use of a family of terpolymers which are amphiphilic and, under physiological conditions, capable of forming micrometric- or nanometric-sized polymer micelles, which consist of acrylic monomers derived from the α-tocopherol molecule and highly hydrophilic monomers such as N-vinyl pyrrolidone and/or N-vinyl caprolactam, vinyl imidazol and other hydrophilic vinyl compounds. This family of copolymers and terpolymers exhibits dual (antioxidant and amphiphilic) activity which offers the possibility of being used as vehicles for other active ingredients with analgesic, anti-inflammatory, tissue regeneration process activating, antibiotic, antiproliferative, antimicrobial effects, etc.

in a first aspect, the present invention relates to a polymer compound of formula (I): 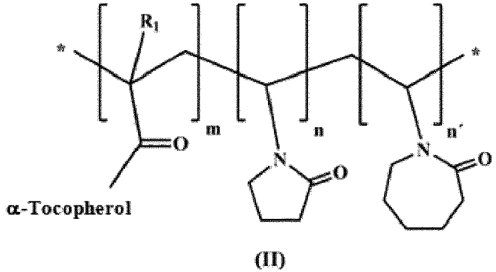
wherein R₁ is selected from hydrogen and linear or branched C₁-C₄ alkyl;
m, n and n' represent the molar fraction of each of the units bound by the symbols [ ] in the final composition of the polymer compound, wherein (m+n+n') = 1
and * is understood as the consecutive repetition of said units.

The three subindeces m, n and n' are other than zero.

In another preferred embodiment, the compound of the invention is the compound of formula (II): 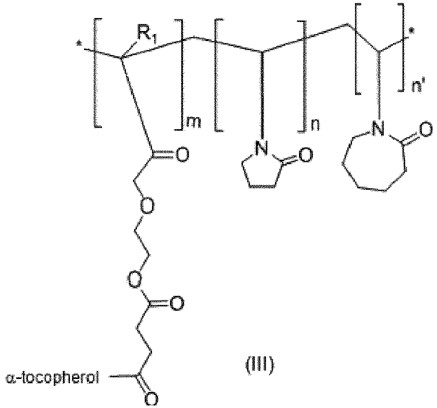 wherein R₁, m, n and n' are as defined above.

in another preferred embodiment, m has a value between 0.10 and 0.15, more preferably 0.15.

in another preferred embodiment, n has a value between 0.00 and 0.85, excluding 0 for the case of a terpolymer, n preferably being between 0.02 and 0.85.

in another preferred embodiment, n' has a value between 0.00 and 0.85, excluding 0 for the case of a terpolymer, n' preferably being between 0.02-0.85. Another aspect of the invention relates to a multi-micellar nanoparticle comprising the polymer compound as defined above.

In a preferred embodiment, the multi-micellar nanoparticle comprises a combination of the copolymers and terpolymers described in the present invention. More preferably, at a weight ratio of 25-50 % terpolymer and 50-75% copolymer.

in another preferred embodiment, the polymer compound of the multi-micellar nanoparticle is the polymer compound of formula (I).

in another preferred embodiment, the polymer compound of the multi-micellar nanoparticle is the polymer compound of formula (II).

in another preferred embodiment, the multi-micellar nanoparticle further comprises a bioactive compound.

in a more preferred embodiment, the bioactive compound is selected from the following group: methylprednisolone, dexamethasone, cortisone, ibuprofen, naproxen, flurbiprofen, ketoprofen, vitamin C, polyphenols, carnosic acid, astaxanthin, vitamin B1, vitamin B6, vitamin B12, ^{®}-carotene derivatives, lutein, allantoin, vitamin A, folic acid, vancomycin, rifampicin, linezolid, quaternary ammonium salts, chlorhexidine.

Another aspect of the invention relates to a controlled release system of bioactive compounds comprising the multi-micellar nanoparticle as described above.

Another aspect of the invention relates to a pharmaceutical composition comprising the controlled release system as described above.

The systems described herein consist of copolymers formed by an acrylic derivative monomer component of α-tocopherol (vitamin E) and a vinyl monomer, which can be, among others, N-vinyl pyrrolidone, N-vinyl caprolactam or a mixture of vinyl compounds that will be an integral part of copolymer systems or terpolymers with a controlled and amphiphilic composition, with a component balance of hydrophilic and hydrophobic sequences that will depend directly on the composition of the systems with the three components, acrylic derivative of vitamin E, N-vinyl pyrrolidone and N-vinyl caprolactam, the incorporation of other vinyl monomers described in the body of the patent furthermore being able to be considered.

The nanoparticulate systems considered in this patent have the property of being autoassociative in physiological conditions to give rise to stable multi-micellar nanoparticles, and furthermore the capacity of being able to dissociate in a reasonable time period, offering a suitable elimination pathway for the systems once they have performed their main function in biomedical applications deemed appropriate.

"Copolymer" in the present invention is understood to mean a macromolecule made up of two different repeating units, referred to as monomers, which can be attached in different ways by means of chemical bonds. The monomers forming the copolymer can be distributed randomly or periodically. Similarly, a "terpolymer" is yielded by the simultaneous polymerization reaction of three monomers that are chemically different in which the ionic, hydrophilic or lipophilic nature of the system as a whole can be modified according to the composition.

The term "amphiphilic copolymer or terpolymer" relates to the systems of two or three different monomers formed by lipophilic sequences or segments attached to hydrophilic segments of varying length.

The polymer compounds of the present invention have the quality of forming nanometric- or micrometric-sized particles, with a characteristic morphology consisting of a hydrophobic core and a hydrophilic shell. They exhibit the remarkable advantage of the size and morphology of the micelles which can be modulated by controlling the molar composition and the concentration of these polymer systems. Micelles with sizes between 50 and 500 nm are readily administrable by injection, in the area where they are required, or applied in aqueous dispersions or hydrophilic gels topically or locally.

The compounds of the present invention can be prepared in a wide range of molar compositions, all of which exhibit good cytocompatibility and suitable micellar stability which allows being used not only as compounds with antioxidant activity per se but also as vehicles for the administration and controlled and targeted release of bioactive components and drugs.

The compounds of formula (I) or (II) as described above can be obtained by a method comprising the following steps:
a) Mixing the alpha-tocopherol with acrylic acid chloride or methacrylic in the presence of a reaction catalyst and activator.
b) Mixing the acrylic monomer obtained in step a) with a hydrophilic monomer, in the presence of a reaction initiator.

Preferably, the catalyst of step a) is a tertiary amine.

Preferably, the reaction time of step a) has a duration comprised between 1 and 60 hours.

Preferably, step a) is performed at a temperature of 15 °C a 60 °C.

Preferably, the initiator of step b) is a radical initiator (organic peroxide, azo compound, perester).

Preferably, the concentration of catalyst used in (a) is between 0.01 and 1.5 equivalents. More preferably, the concentration of reaction activator used in (a) is between 1.0 and 1.8 equivalents.

Preferably, the concentration of monomer used in step (b) is between 0.1 and 10 M. More preferably, the concentration of initiator used in step (b) is between 0.01 and 0.1 M.

The multi-micellar nanoparticles described above can be obtained by a method in which the polymer compound of formula (II or III) is dissolved in an organic solvent and nanoprecipitated in an aqueous medium or physiological medium miscible with the organic solvent.

Preferably, the organic solvent is miscible with water and is selected from the following group: dioxane, tetrahydrofuran, dimethylsulfoxide, and dimethylformamide.

Preferably, the organic solvent is at a concentration of between 1 and 40 mg/ml. in the mixture.

Preferably, the aqueous medium is at a concentration of between 0.2 and 10 mg/ml. in the mixture.

Preferably, a mixture of poly(MVE-*co*-VP) (15:85) (molar feed ratio) copolymer and terpolymers is used, represented by formulas (I) and (II).

Throughout the description and the claims, the word "comprises" and its variants do not intend to exclude other technical features, additives, components or steps. For those skilled in the art, other objects, advantages and features of the invention may be inferred from both the description and the embodiment of the invention. The following examples and figures are provided by way of example and are not intended to limit the present invention.

### BRIEF DESCRIPTION OF THE FIGURES

FIG. 1 shows the synthesis diagram of the methacrylic monomer derived from the α-tocopherol molecule, MVE. Similar diagrams can be proposed for the corresponding acrylic derivatives AVE, ATOS, MTOS.
FIG. 2 shows the ¹H-NMR spectrum (400 MHz, CDCl3) of the methacrylic monomer derived from the α-tocopherol molecule (MVE).
FIG. 3 shows the ¹H-NMR spectrum (400 MHz, CDCl3) of the poly(MVE-co-VP) copolymer (15:85 molar feed ratio, Cop-85).

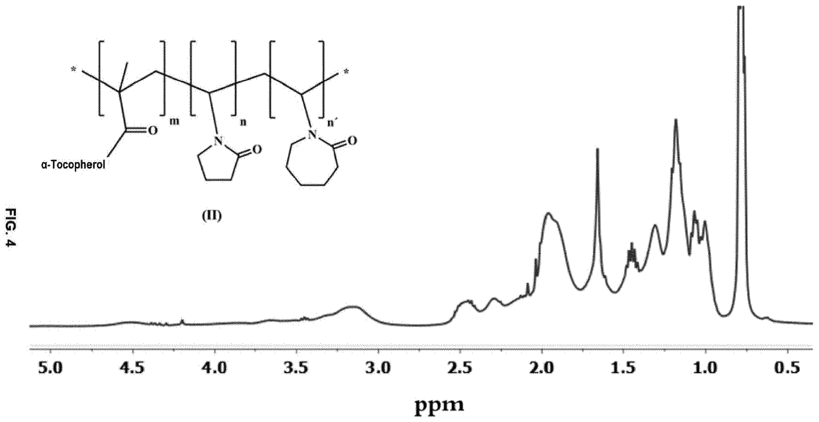

FIG. 4 shows the ¹H-NMR spectrum (400 MHz, CDCl3) of the poly(MVEco-VP-co-VC) terpolymer with molar composition feed ratio (15:83:2).
FIG. 5 shows scanning electron microscopy (SEM) (a) and atomic force microscopy (AFM) (b) images of nanoparticles obtained with methacrylic copolymers and terpolymers derived from the α-tocopherol molecule *and N-*vinylpyrrolidone (VP) and N-vinyl caprolactam poly(MVE-co-VP-co-VC).
FIG. 6 shows the results of the viability assay of human fibrobroblasts exposed to the nanoparticles obtained from the poly(MVE-co-VP) copolymers and poly(MVE-co-VP-co-VC) terpolymers. AlamarBlue assay, mean relative cell viability ± standard deviation (n=8; *: p<0.05) are represented.
FIG. 7 shows of the comparison of the particle size distributions obtained from the control poly(MVE-co-VP) copolymer throughout 4 weeks of conservation in the refrigerator at 4 °C.
FIG. 8 shows the comparison of the particle size distributions obtained from the control poly(MVE-co-VP) copolymer throughout 4 weeks of conservation in the freezer at -20 °C.
FIG. 9 shows the comparison of the particle size distributions obtained from the control poly(MVE-co-VP) copolymer after lyophilizing and keeping the particles under magnetic stirring for 2 h.
FIG. 10 shows the comparison of the particle size distributions obtained from the control poly(MVE-co-VP) copolymer incorporating TPGS as a surfactant.

### EXAMPLES

Next, the invention will be illustrated by means of assays carried out by the inventors that demonstrate the effectiveness of the product of the invention.

### Example 1

### Synthesis of a methacrylic monomer derived from the α-tocopherol molecule (MVE)

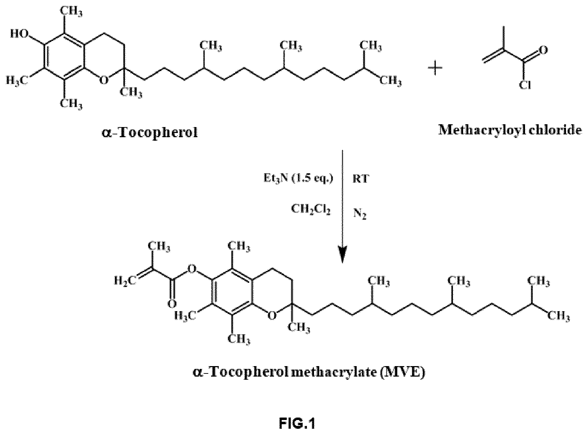

The monomer was prepared by reacting the α-tocopherol molecule with methacryloyl chloride in the presence of triethylamine. Dichloromethane was used as a solvent. The reaction diagram is shown in Figure 1.

The α-tocopherol molecule (1 equivalent) and triethylamine (1.5 equivalents) in 150 ml of dichloromethane were introduced in a round-bottom flask. The methacryloyl chloride (1.2 equivalents) was added slowly, dropwise, with constant stirring under nitrogen atmosphere and using an ice bath. The reaction mixture was kept for 24 hours at room temperature. After 24 hours, the reaction mixture was washed by successive extractions with 1N solutions of NaOH and HCl and the solvent was subsequently removed at reduced pressure. The resulting medium was redissolved in hexane and washed 3 times with the same solution of HCl used previously for the purpose of improving purification efficacy. The overall efficiency of the reaction was greater than 90 %.

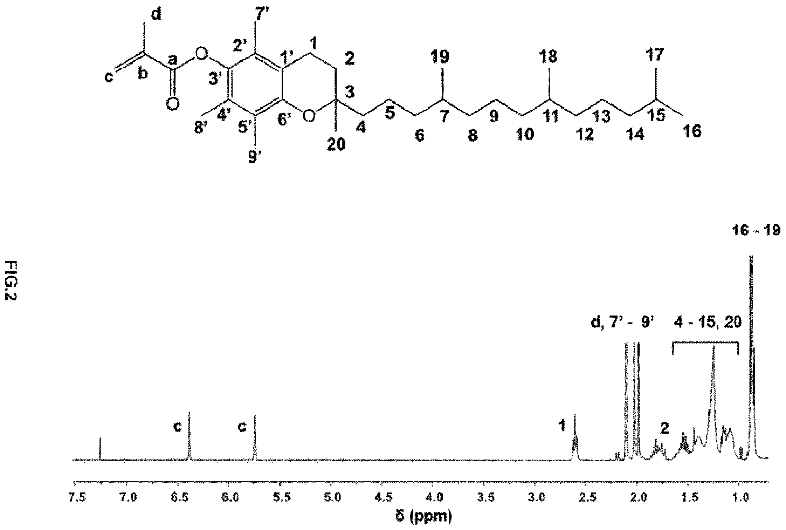

Figure 2 shows the spectrum of the MVE with the assignation of the resonance signals verifying correct synthesis of the monomer. Signals corresponding to the vinyl protons and to the protons of group (CH₃) of the acrylic group can clearly be observed in the spectrum.

### Synthesis of the copolymer carrying the poly(MVE-co-VP) alpha-tocopherol molecule (not according to the invention)

Copolymers were prepared by reacting the obtained MVE monomer and N-vinylpyrrolidone (VP) as a hydrophilic monomer from a composition in the MVE:VP feed ratio (%-molar) of 15:85.

The copolymer was prepared by high-conversion radical polymerization. The reaction was carried out by dissolving the monomers in dioxane (1 M) using 2,2'-Azobisisobutyronitrile (AIBN) as initiator (1.5×10⁻² M). The prepared solution was deoxygenated by a stream of N2 (g) for 30 minutes at room temperature. The reaction mixture was maintained at 60 °C inside a temperature-controlled oven for 24 hours. Finally, the obtained product was purified by dialysis and lyophilized such that an amorphous white powder was obtained with the following parameters:
- Composition feed ratio F_{MVE}= 0.15;
- Composition in the copolymer f_{MVE}= 0.40;
- Yield, % by weight =59;
- Weight average molecular weight M_{w} ·10⁻³= 29.3;
- Number average molecular weight Mₙ · 10⁻³ = 18;
- Polydispersity index Pdl = 1.9.

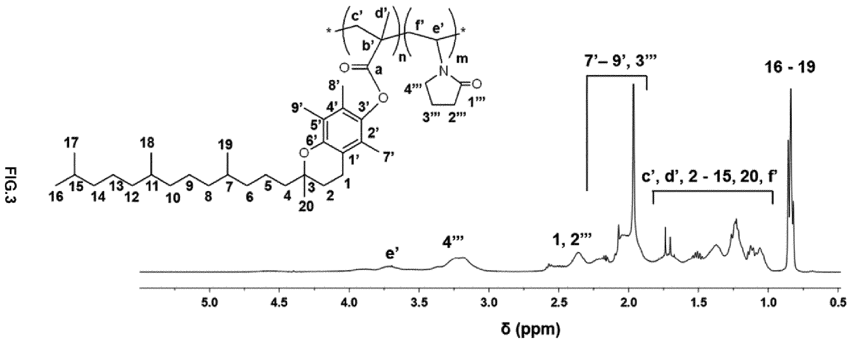

The molar composition of the prepared copolymers was calculated from their corresponding ¹H-NMR spectra. Figure 3 shows the spectrum of the copolymer prepared from molar fractions in the feed ratio of MVE:VP 15:85. The disappearance of the signals characteristic of the vinyl protons is observed. Furthermore, the broadening of the signals as a consequence of polymerization, and with it, the macromolecular nature of the synthesized polymers, can be seen.

To calculate the molar compositions of the copolymer, the values of the normalized integrals of the signals characteristic of each monomer are considered. Specifically, the signals taken into account are those appearing in the range of 3 - 5 ppm corresponding to 3 protons of VP and 4 protons of MVE (CH2-13 and CH2-14) and the signal at 0.86 ppm corresponding to 12 protons of MVE (CH3-4a', CH3-8a', CH3-12a' and CH3-13'.

The weight average molecular weights (Mw), number average molecular weights *(Mn)* and the polydispersity index (*Ð*) are obtained by size-exclusion chromatography (SEC). To that end, a Perkin-Elmer chromatograph equipped with an LC-250 Series isocratic pump, connected to a 200 Series refractive index detector, was used. The samples were eluted using three polystyrene-divinylbenzene columns (Polymer Laboratories) connected in series with a pore size of 10³, 10⁴ and 10⁵ A at 30 °C. Tetrahydrofuran (THF) was used as the eluent at a flow of 1 ml/min. Poly(methyl methacrylate) standards with a molecular weight between 10,300 and 1,400,000 Da were used for calibration.

To know the microstructural organization of the polymer chains and the reactivity of each of the monomers, the monomer reactivity ratios were calculated by quantitative analysis of ¹H-NMR in situ.

Copolymerization was carried out in NMR equipment using deuterated dioxane as a solvent at 60 °C. The reactions were performed in the resonance tube after deoxygenating with nitrogen and placing in a capillary tube with dichlorobenzene which will serve as the reference signal. Different concentrations of comonomers were studied for the purpose of covering the entire range of concentrations, with the total monomer concentration being 0.25 M.

This method finds that MVE is considerably more reactive than VP (rVP = 0.03 and rMVE = 2.20) and, therefore, the large difference in reactivity of the monomers used. This gives rise to macromolecular chains with long sequences of both monomers and few chains of an intermediate composition. This compositional gradient throughout the macromolecular chains together with the different hydrophilia of the monomers gives rise to self-assembled polymer structures of the multi-micellar type.

### Example 2

### Preparation of terpolymers derived from MVE, VP and VC: poly(MVE-co-VP-co-VC)

Using the same methodology described in Example 1, vitamin E methacrylate (MVE), N-vinylpyrrolidone (VP) and *N-*vinyl caprolactam (VC) terpolymers have been prepared with the molar compositions in the feed ratio summarized in Table 1. The synthesized terpolymers are characterized by ¹H-NMR and SEC to know their actual composition and molecular weight, obtaining the results compiled in Table 1.

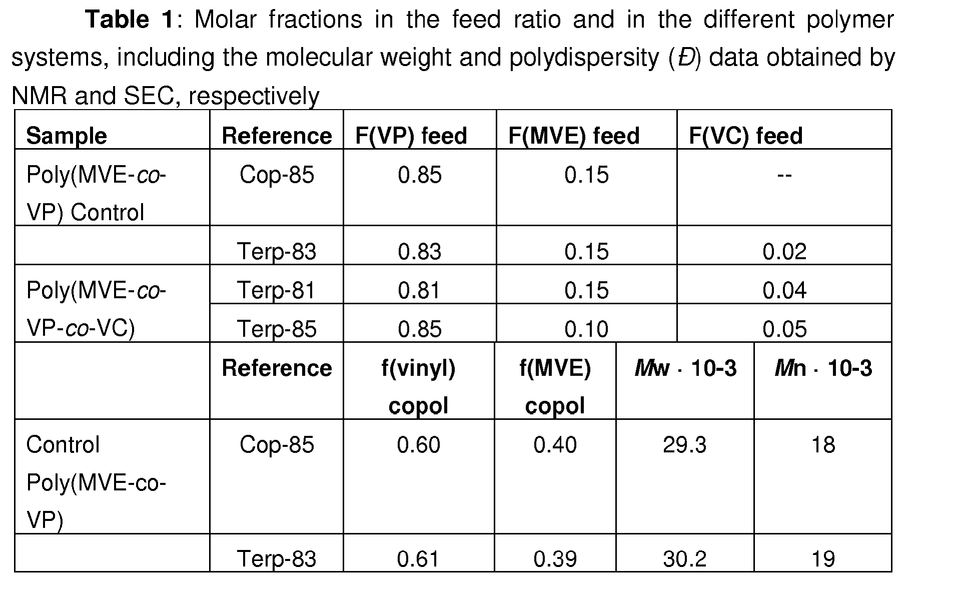

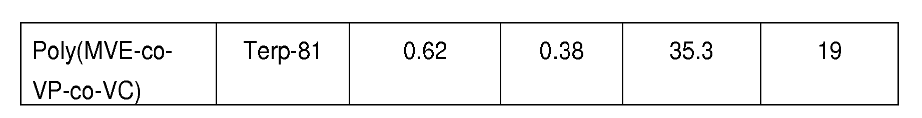

### Example 3

### Preparation of nanoparticles from poly(MVE-co-VP) copolymers and 15 poly(MVE-co-VP-VC) terpolymers.

Non-loaded nanoparticles were prepared from the poly(MVE-co-VP) copolymers (comparative) and poly(MVE-co-VP-co-VC) terpolymers (according to the invention), synthesized by the nanoprecipitation method. To that end, the copolymers and terpolymers 20 (10 mg/ml) were dissolved in a water-miscible organic solvent, using dioxane in this case, although it is possible to use other solvents miscible with aqueous media such as dimethylsulfoxide, dimethylformamide, tetrahydrofuran or mixtures thereof. Next, this solution was added dropwise and with vigorous stirring to the necessary amount of the aqueous medium to obtain a concentration of nanoparticles between 0.01 and 10 mg/ml. For the cellular assays, the nanoparticle solutions were prepared under different conditions described in the following sections.

The nanoparticle size distribution was determined by dynamic light scattering (DLS) using Zetasizer Nano ZS equipment (Malvern Instruments) equipped with a He-Ne laser at 633 nm and with an angle of 173°. The measurements were taken in polystyrene cuvettes at room temperature. The particle sizes obtained, as well as the polydispersity for the NPs obtained from previously synthesized copolymers and terpolymers, are compiled in Table 2. In all cases, the particle size ranges between 130 and 185 nm with low polydispersity values.

**Table 2: Hydrodynamic diameter (Dh) and polydispersity (PDI) of the nanoparticles obtained from the synthesized Poly(MVE-co-VP) copolymers and Poly(MVEco-VP-co-VC) terpolymers**

| **NP** | ***D*h (nm)** | **PDI** |
|---|---|---|
| Cop-85 | 136 ± 6 | 0.14 ± 0.04 |
| Terp-83 | 156 ± 1 | 0.12 ± 0.02 |
| Terp-81 | 171 ± 1 | 0.14 ± 0.03 |
| Terp-85 | 183 ± 1 | 0.15± 0.02 |

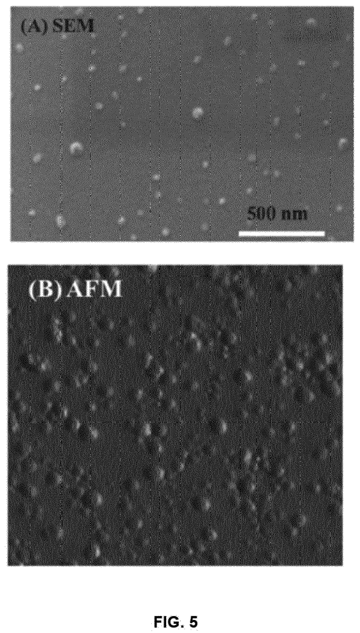

The morphology of the nanoparticles was observed by Scanning Electron Microscopy (SEM, Philips XL 30 ESEM) and Atomic Force Microscopy (AFM) using the tapping mode. To that end, a drop of the nanoparticle suspension at a 1:50 dilution was deposited and left to dry for 24 hours on a 14 mm glass disc. Figure 5 includes the obtained images such that it can be seen that the particles are spherical and do not tend to aggregate.

The viability assays were performed using human fibroblasts which were maintained and multiplied at 37 °C in an atmosphere with 5 % CO2, using as culture medium DMEM, supplemented with 10 % fetal bovine serum, 1 % of a solution of penicillin-streptomycin and 2 % L-glutamine.

The viability assay was carried out by an AlamarBlue assay and different dilutions of the NPs in PBS, assaying concentrations between 2 and 0.06 mg/ml. Specifically, the AlamarBlue reagent is a redox indicator that changes color in response to the chemical reduction of the culture medium that takes place as a consequence of cell growth and proliferation.

After a 24 h cell growth period in an atmosphere with 5 % CO₂, the culture medium was changed with 50 µl of the nanoparticles solutions that were kept in contact with the cells for 24 hours under the same conditions. Once this time period ended, the content of the wells was removed and a solution of the AlamarBlue reagent was added and left to act for 3 hours. Finally, fluorescence readings were taken at 460 /630 em/ex.

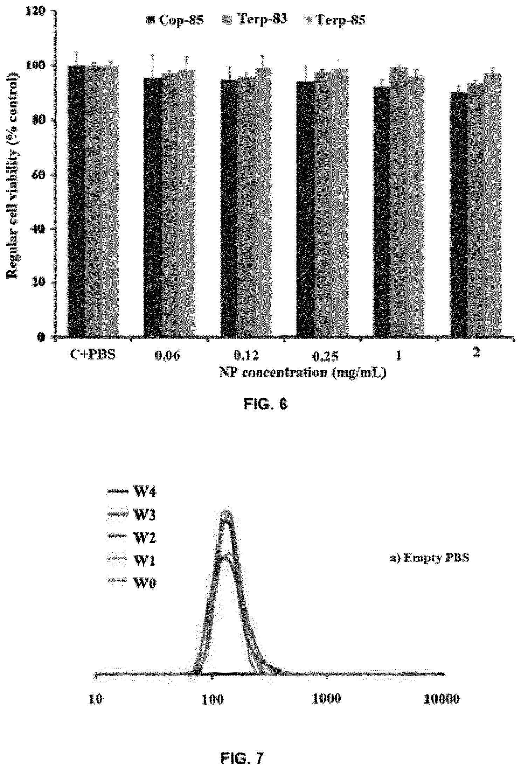

Figure 6 shows the cell viability results for the NPs prepared from the control poly(MVE-co-VP) copolymer and the different poly(MVE-co-VP-co-VC) terpolymers. In view of the represented results, none of the NP concentrations is cytotoxic, maintaining cell viability above 90 % without significant differences with respect to the control.

### Example 4

*Preparation of nanoparticles loaded with dexamethasone from poly(MVE-co-VP) copolymers* (comparative) *and poly(MVE-co-VP-co-VC)* terpolymers (according to the invention).

One of the most appealing factors of the prepared nanoparticles is the possibility that they can be used in a very effective manner as vehicles of bioactive compounds that are insoluble or slightly soluble in aqueous media. The following experiments demonstrate the capacity of the nanoparticles prepared with copolymers and terpolymers considered in this patent. Although the results have been limited to the dexamethasone load as an active compound with anti-inflammatory activity, the copolymer and terpolymer nanoparticles can be loaded and used as vehicles for a wide range of bioactive compounds and medicinal products, with the sole condition that they must be insoluble or slightly soluble in aqueous media or physiological medium, whereby offering a very suitable route for the application in the human body of systems which have an absorption limit due to their scarce solubility in physiological medium. The application is not limited to a specific family of bioactive components or medicinal products that are difficult to administer or have a limited administration. This opens up a very appealing scenario for localized, targeted and sustained administration by any route of a wide array of possibilities, with its advantage from a therapeutic viewpoint.

The loaded nanoparticles were prepared at a concentration of 2 mg/ml in PBS and in NaCl from a copolymer in dioxane concentration of 50 mg/ml. Additionally, a concentration of 15 % by weight with respect to the polymer of the active molecule, dexamethasone (Dx) in this case, was added. The method is similar to that described for the non-loaded NPs, such that the organic phase was left to drip on the aqueous (PBS or 100 mM NaCl), to finally dialyze the resulting solution for 3 days.

To calculate the encapsulation efficiency (EE) of dexamethasone, the different prepared NPs were lyophilized to eliminate the aqueous phase, obtaining a white powder with a yield of about 90 %. Next, 5 mg of each formulation were dissolved in chloroform for 24 hours, to then add 2 ml of ethanol and leave stirring for 24 hours until observing precipitation of the polymer. The obtained samples were centrifuged at 10,000 rpm and the supernatant was analyzed by UV (NanoDrop One Thermo Scientific) at a wavelength of 239 nm corresponding to dexamethasone.

The nanoparticle size distribution could be determined by dynamic light scattering (DLS) using Zetasizer Nano ZS equipment (Malvern Instruments) equipped with a Helium-Neon laser at 633 nm and with detection at an angle of 173°. The measurements were taken in polystyrene cuvettes at room temperature.

Table 3 compiles the sizes, polydispersity and EE of the particles loaded with *Dx,* obtained from the poly(MVE-co-VP) copolymers, poly(MVE-co-VP-co-VC) terpolymers and different mixtures of both polymer systems.

**Table 3: Hydrodynamic diameter (Dh) and polydispersity (PDI) of the nanoparticles loaded with dexamethasone obtained from synthesized Poly(MVE-co-VP) copolymers and Poly(MVE-co-VP-co-VC) terpolymers as well as different mixtures.**

| **NP** | **Drug** | ***EE*** | ***D*ₕ (nm)** | **PDI** |
|---|---|---|---|---|
| Cop-85 | Dexamethasone | 55 | 140 ± 7 | 0.10 ± 0.04 |
| Terp-83 | | 45 | 154 ± 1 | 0.13 ± 0.02 |
| Terp-81 | | 42 | 180 ± 2 | 0.19 ± 0.02 |
| Terp-85 | | 40 | 154 ± 1 | 0.10 ± 0.03 |
| Cop-85 + Terp-83 (50:50 w:w) | | 42 | 164 ± 2 | 0.14 ± 0.02 |
| Cop-85 + Terp-83 (75:25 w:w) | | 48 | 167 ± 1 | 0.08 ± 0.01 |
| Cop-85 + Terp-85 (50:50 w:w) | | 48 | 149 ± 1 | 0.08 ± 0.02 |
| Cop-85 + Terp-85 (75:25 w:w) | | 47 | 172 ± 1 | 0.21 ± 0.01 |

The size distribution obtained for the NPs loaded with dexamethasone from the poly(MVE-co-VP) copolymers, poly(MVE-co-VP-co-VC) terpolymers and mixtures of both polymer systems is in a range of 140 to 170 nm, with low polydispersities and unimodal size distributions.

### Example 5

### Study of the stability of the different nanoparticles obtained from poly(MVE-co-VP) copolymers and poly(MVE-co-VP-co-VC) terpolymers.

To study the effect that the method of storage may have on the size stability of multi-micellar nanoparticles, these were cooled in a refrigerator, frozen at -20 °C and lyophilized, as described below.

### • Cooling in a refrigerator:

For 4 continuous weeks (1 month), the size distribution of the empty control NPs and NPs loaded with Dx and obtained from the poly(MVEco-VP) copolymers was measured. The obtained results, Dh and PDI, throughout each week are compiled in Table 4.

**Table 4: Particle size and polydispersity development whilst kept in a refrigerator at 4 °C of the control nanoparticles obtained from poly(MVE-co-VP) copolymers.**

| **Sample** | **Load** | **Week 0 (W0)** | | **Week 1 (W1)** | | **Week 2 (W2)** | | **Week *3 (W3)*** | | **Week 4 (W4)** | |
|---|---|---|---|---|---|---|---|---|---|---|---|
| | | **Dₕ (nm)** | **PDI** | **Dₕ (nm)** | **pdl** | **Dₕ (nm)** | **PDI** | **(nm)** | **PDI** | **Dₕ (nm)** | **PDI** |
| Cop-85 PBS | Non-loaded | 138 ±6 | 0.14 ± 0.04 | 137±5 | 0.08 ± 0.04 | 140±1 | 0.17± 0.02 | 139 ± 2 | 0.11 ± 0.02 | 144±5 | 0.16 ± 0.05 |
| | Dx 15 % | 145±7 | 0.10 ± 0.04 | 138±8 | 0.10 ± 0.02 | 145±5 | 0.13 ± 0.04 | 139 ± 8 | 0.14 ± 0.02 | 140 ± 9 | 0.12± 0.01 |
| Cop-85 NaCl | Non-loaded | 135 ±5 | 0.09 ± 0.01 | 136±8 | 0.12 ± 0.03 | 136±7 | 0.12 ± 0.01 | 138 ± 10 | 0.12± 0.06 | 142 ± 11 | 0.16 ± 0.05 |
| | Dx 15 % | 138±5 | 0.10 ± 0.01 | 134 ± 7 | 0.10 ± 0.03 | 136±6 | 0.11 ± 0.03 | 135±8 | 0.10 ± 0.01 | 134±7 | 0.12± 0.02 |

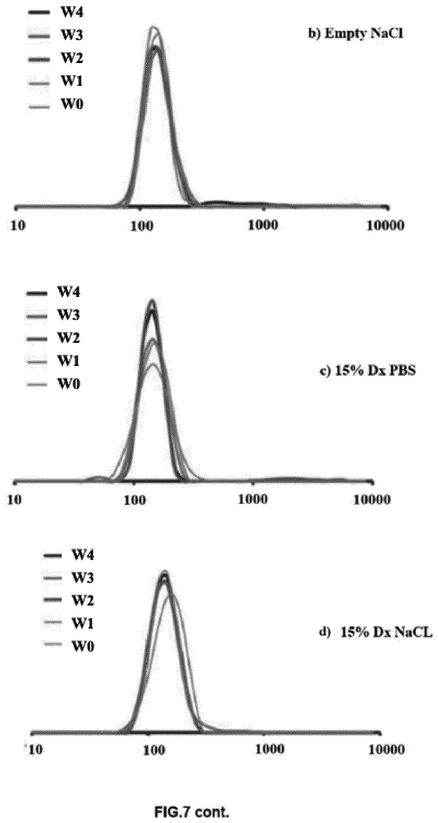

Particle size distributions for empty and loaded particles, prepared both in PBS and in NaCl, are compared in Figure 7. The initial results (W0, recently prepared NPs) as well as the curves after each week of storage at 4 °C are shown. The samples measured in each week are called W1 (week 1), W2 (week 2), W3 (week 3) and W4 (week 4).

Table 4 and Figure 7 show the size distribution obtained after 4 weeks of storage of the NPs in refrigerator (4 °C) for the different NPs. It can be observed that the results are similar for the 4 weeks of storage in addition to being similar to the original distribution sizes (week 0) for all the NP types, in a general manner. This behavior is analogous to that observed with the NPs obtained from different synthesized poly(MVE-co-VP-co-VC) terpolymers.

For 4 weeks, the control NP size distribution was measured to observe their behavior after being stored under freezing at -20 °C. The measurement protocol was carried out by continuous weeks of freezing (1, 2, 3 and 4 weeks), that is, 4 different samples were used, each one being thawed and thawed at different times.

Next, the size distribution obtained for each NP type for four weeks, including the respective polydispersities (PDI), can be observed in Table 5. As a result, stable NPs are observed, where size variations are not significant, with low polydispersity values.

**Table 5: Particle size and polydispersity development whilst kept in the freezer at -20 °C of the control nanoparticles obtained from poly(MVE-co-VP) copolymers.**

| **Sample** | **Load** | **Week 0 (W0)** | | **Week** 1 **(W1)** | | **Week 2 (W2)** | | **Week 3 (W3)** | | **Week 4 (W4)** | |
|---|---|---|---|---|---|---|---|---|---|---|---|
| | | **Dh (nm)** | **PDI** | **Dh (nm)** | **PDI** | **Dh (nm)** | **PDI** | **Dh (nm)** | **PDI** | **Dh (nm)** | **PDI** |
| Cop-85 PBS | Non-loaded | 138 ± 6 | 0.14 ± 0.04 | 137±1 | 0.11 ± 0.01 | 135±1 | 0.12 ± 0.02 | 132 ± 1 | 0.12 ± 0.01 | 151 ±4 | 0.15 ± 0.02 |
| | Dx 15% | 145 ± 7 | 0.10 ± 0.04 | 147 ±2 | 0.11 ± 0.01 | 143 ± 6 | 0.10 ± 0.01 | 138 ± 10 | 0.11±0.02 | 141 ±4 | 0.09 ± 0.02 |
| Cop-85 NaCl | Non-loaded | 135 ± 5 | 0.09 ± 0.01 | 139±1 | 0.10 ± 0.01 | 131 ± 2 | 0.11 ± 0.01 | 134 ± 5 | 0.08 ± 0.02 | 132 ±2 | 0.23 ± 0.03 |
| | Dx 15% | 138 ± 5 | 0.10 ± 0.01 | 135 ±7 | 0.11 ± 0.02 | 143±1 | 0.10 ± 0.01 | 134 ± 13 | 0.12 ± 0.06 | 143 ±2 | 0.13 ± 0.02 |

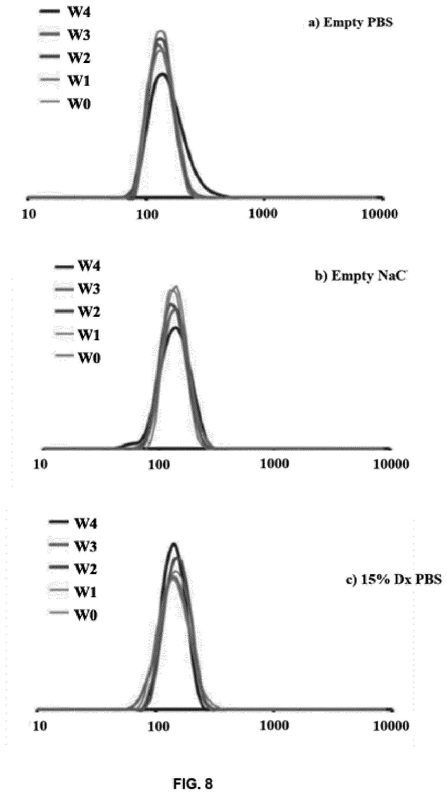

Figure 8 (a) distribution of NPs in PBS with no load, b) NaCl with no load, c) 15% dexamethasone in PBS, and d) 15 % dexamethasone in NaCl, respectively), shows the size distribution of the synthesized NPs, measured for different freezing times, as mentioned above.

The graphs show that the obtained curves are similar to the sizes of week 0, and the overlap of the curves corresponding to each week of freezing can also be observed. This behavior is identical for all synthesized NP types, both for those obtained from poly(MVE-co20 VP) copolymers and poly(MVE-co-VP-co-VC) terpolymers. Therefore, it can be said that all the NPs are stable after a month of storage under the detailed conditions. Furthermore, the formation of agglomerates is not observed since there is an absence of secondary peaks in the curves.

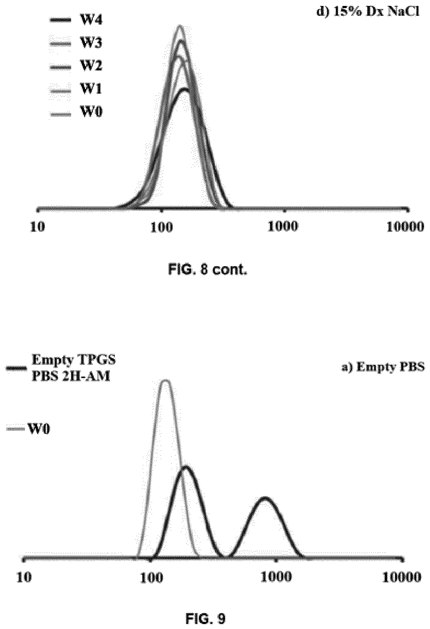

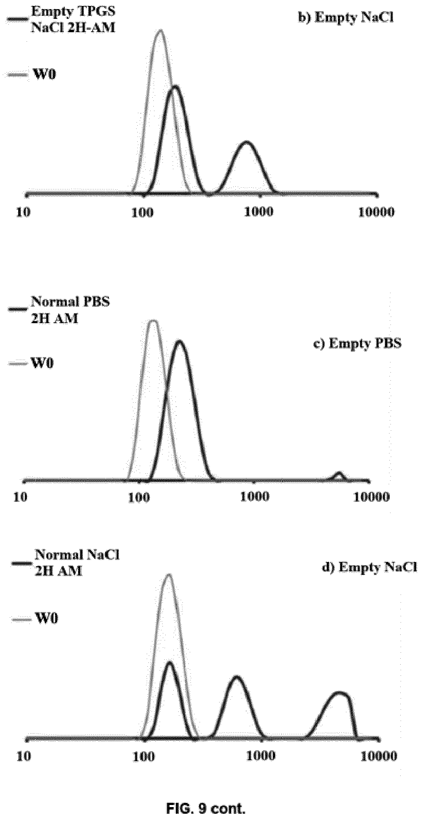

3 ml of each type of nanoparticles were lyophilized for 2 days, to then be resuspended in the same volume of ultrapure Milli-Q water. Subsequently, the size distribution was measured after 2 hours of magnetic stirring at a speed of 1000 rpm and 5 or 10 minutes under ultrasound application (Sonics Vibra-Cell) at 29 % amplitude (1/2W/tip) in an ice bath. The obtained results are compiled in Table 6 and the particle size distributions are compared in Figure 9.

In view of the results compiled in Table 6, size distribution after 2 hours of magnetic stirring are larger than the original diameters, while additionally showing second peaks with diameters of larger sizes, and for that reason 5 minutes of ultrasound was applied to obtain the desired diameters.

It can be seen in Table 6 that after 5 minutes of ultrasound, there is a presence of second peaks and size distribution greater than the originals (Week 0), and for that reason ultrasound needed to be applied another 10 minutes, the secondary peaks do not disappear, but they do decrease, in terms of size distribution, though they are slightly larger (around 180 nm), they approach the original diameters (about 150 nm). These results show that magnetic stirring alone is not enough after resuspending, and the application of ultrasounds for at least 10 minutes is necessary.

For the purpose of eliminating the use of the ultrasound and using magnetic stirring alone after lyophilizing, NPs were synthesized again incorporating a stabilizing agent, TGPS or α-tocopherol polyethylene glycol 1000-succinate (R14-M043 Sigma-Aldrich) by two different methods:
1. Incorporating 0.02 %wt TPGS in the synthesis of NPs, with respect to the entire volume (only NPs loaded with 15 % dexamethasone were synthesized).
2. Resuspending in a solution of ultrapure water (Milli-Q) with 0.02 % TGPS. The results are shown below.

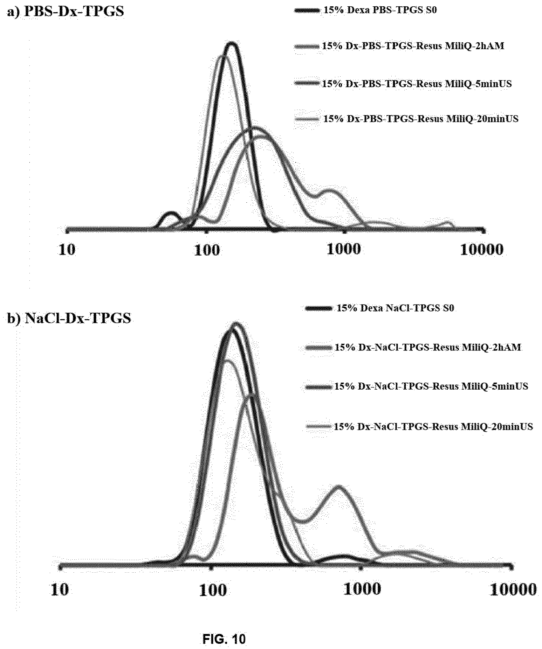

It can be seen in all the cases in Figure 10 that 2 hours of magnetic stirring was not enough to obtain the original diameters, despite incorporating a certain amount of TPGS. For this reason, ultrasound was applied (under the same conditions specified above) for 5 minutes; nonetheless, the size distribution was much greater than the original before resuspending, to that end the NPs were subjected to another 20 minutes of ultrasound, where a size distribution very close to the original is observed in all cases.

**Table 6: Particle size and polydispersity after lyophilizing control nanoparticles obtained from poly(MVE-co-VP) copolymers and subjecting them to a magnetic stirring or ultrasounds (US).**

| **Time** | **Sample** | **Load** | **Dₕ** 1 **(nm)** | **Dₕ 2 (nm)** | **PDI** |
|---|---|---|---|---|---|
| 2h Magnetic stirring | Cop-85 PBS | Non-loaded | 238 ±20 | 944 ±29 | 0.41 ± 0.01 |
| | | Dx 15 % | 149 ±14 | 552 ± 88 | 0.70 ± 0.06 |
| | Cop-85 NaCl | Non-loaded | 182 ±20 | 779 ± 204 | 0.46 ± 0.06 |
| | | Dx 15% | 194 ±5 | 710 ±57 | 0.38 ±0.01 |
| 5 min US | Cop-85 PBS | Non-loaded | 200 ±29 | 174S ± 730 | 0.214 ±0.005 |
| | | Dx 15 % | 383 ±89 | 857 ± 43 | 0.34 ± 0.02 |
| 10 min US | Cop-85 NaCl | Non-loaded | 195 ±8 | - | 0.11 ±0.01 |
| | Cop-85 PBS | Non-loaded | 173 ±7 | 388 ±51 | 0.160 ±0.004 |
| | | Dx 15 % | 186 ±6 | 1776 ±151 | 0.12 ±0.03 |
| | Cop-85 NaCl | Non-loaded | 182 ±5 | - | 0.11 ±0.02 |

Finally, it can be confirmed that the addition of TPGS, by either of the two methods does not significantly improve the original size distribution obtained after resuspending and subjecting the NPs to 2 hours of magnetic stirring, where it is 5 necessary to apply ultrasound for at least 20 minutes.

### Example 6

### Scaling in the preparation of control polymer nanoparticles obtained from poly(MVE-co-VP) copolymers

For the purpose of synthesizing larger volumes of NPs, the latter were synthesized using the same protocol, but at higher NP stirring speeds. The size distribution obtained for 30 and 50 ml of NPs are compiled in Table 7.

**Table 7: Particle size and polydispersity after scaling in the preparation of the control nanoparticles obtained from poly(MVE-co-VP) copolymers.**

| **Sample** | | **Volume (ml)** | **Dh (nm)** | **PDI** |
|---|---|---|---|---|
| Cop-85-PBS | Non-loaded | 30 | 145 ± 1 | 0.10 ± 0.01 |
| | | 50 | 145 ± 2 | 0.09 ± 0.01 |
| | 15% DX | 30 | 146 ±1 | 0.07 ± 0.01 |
| | | 50 | 148 ± 1 | 0.10 ± 0.02 |
| Cop-85-NaCl | Non-loaded | 30 | 138 ± 1 | 0.07 ± 0.01 |
| | | 50 | 144 ± 1 | 0.09 ± 0.02 |
| | 15% DX | 30 | 123 ± 1 | 0.11 ± 0.03 |
| | | 50 | 136 ± 2 | 0.09 ± 0.02 |

After scaling the preparation of the NPs, it can be observed that the size distribution obtained is similar at a larger volume, so it could be said that the 5 protocol for the preparation of NPs is valid with larger volumes, with the only variation of increasing the stirring speed for the synthesis of NPs.

## Claims

1. The terpolymer of formula (I): 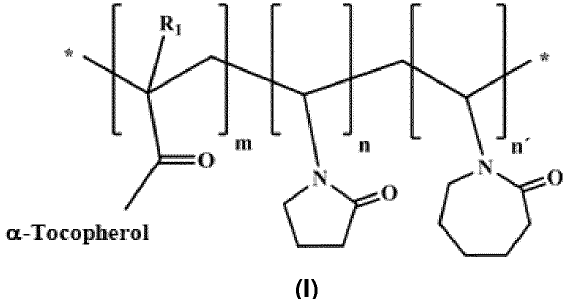
wherein R₁ is selected from hydrogen and linear or branched C₁-C₄ alkyl;
m, n and n' represent the molar fraction of each of the units bound by the symbols [ ] in the final composition of the polymer compound, wherein (m+n+n') = 1 and m, n and n' are other than zero,
and * is understood as the consecutive repetition of said units.

2. The terpolymer of formula (II): 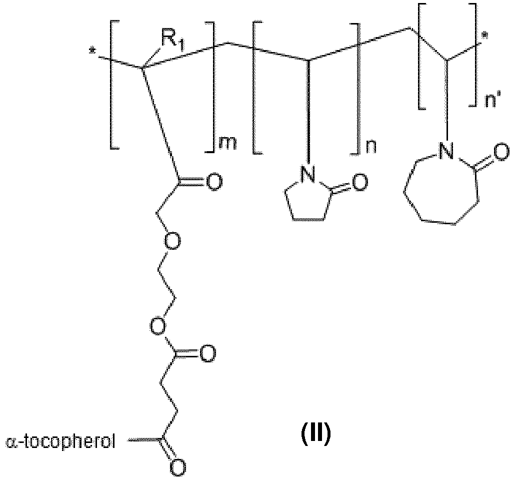 wherein R₁, m, n and n' are as defined in claim 1.

3. The terpolymer according to any of the preceding claims, wherein m has a value of 0.15.

4. A multi-micellar nanoparticle comprising the terpolymer according to any of claims 1 to 3.

5. The multi-micellar nanoparticle according to claim 4, wherein said terpolymer is the compound of formula (I).

6. The multi-micellar nanoparticle according to claim 4 wherein said terpolymer is the compound of formula (II).

7. The multi-micellar nanoparticle according to claim 4, further comprising a bioactive compound.

8. The multi-micellar nanoparticle according to claim 7, wherein the bioactive compound is selected from the following group: methylprednisolone, dexamethasone, cortisone, ibuprofen, naproxen, flurbiprofen, ketoprofen, vitamin C, polyphenols, carnosic acid, astaxanthin, vitamin B1, vitamin B6, vitamin B12, β-carotene derivatives, lutein, allantoin, vitamin A, folic acid, vancomycin, rifampicin, linezolid, quaternary ammonium salts, chlorhexidine.

9. A controlled release system of bioactive compounds comprising the multi-micellar nanoparticle according to any of claims 4 to 8.

10. A pharmaceutical composition comprising the controlled release system according to claim 9.

## Patentansprüche

1. Terpolymer der Formel (I): 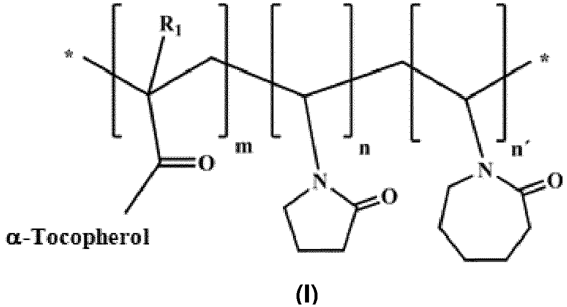
wobei R₁ aus Wasserstoff und linearem oder verzweigtem C₁-C₄-Alkyl ausgewählt ist; m, n und n' den Stoffmengenanteil jeder der durch die Symbole [ ] gebundenen Einheiten in der endgültigen Zusammensetzung der Polymerverbindung darstellt, wobei (m+n+n') = 1 und m, n und n' ungleich Null sind,
und * als die aufeinanderfolgende Wiederholung der genannten Einheiten verstanden wird.

2. Terpolymer der Formel (II): 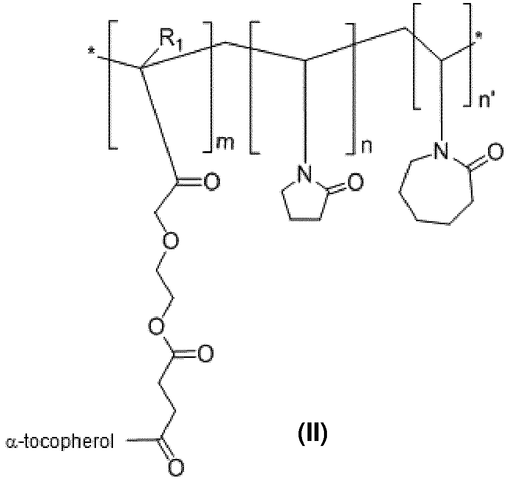 wobei R₁, m, n und n' wie in Anspruch 1 definiert sind.

3. Terpolymer gemäß einem der vorhergehenden Ansprüche, wobei m einen Wert von 0,15 hat.

4. Multimizellen-Nanopartikel, umfassend das Terpolymer nach einem der Ansprüche 1 bis 3.

5. Multimizellen-Nanopartikel gemäß Anspruch 4, wobei das Terpolymer die Verbindung der Formel (I) ist.

6. Multimizellen-Nanopartikel gemäß Anspruch 4, wobei das Terpolymer die Verbindung der Formel (II) ist.

7. Multimizellen-Nanopartikel gemäß Anspruch 4, ferner umfassend eine bioaktive Verbindung.

8. Multimizellen-Nanopartikel gemäß Anspruch 7, wobei die bioaktive Verbindung aus der folgenden Gruppe ausgewählt ist: Methylprednisolon, Dexamethason, Cortison, Ibuprofen, Naproxen, Flurbiprofen, Ketoprofen, Vitamin C, Polyphenolen, Carnosolsäure, Astaxanthin, Vitamin B1, Vitamin B6, Vitamin B12, β-Carotin-Derivate, Lutein, Allantoin, Vitamin A, Folsäure, Vancomycin, Rifampicin, Linezolid, quartären Ammoniumsalzen, Chlorhexidin.

9. System zur kontrollierten Freisetzung bioaktiver Verbindungen, umfassend den Multimizellen-Nanopartikel gemäß einem der Ansprüche 4 bis 8.

10. Pharmazeutische Zusammensetzung, umfassend das System zur kontrollierten Freisetzung nach Anspruch 9.

## Revendications

1. Terpolymère de formule (I) : 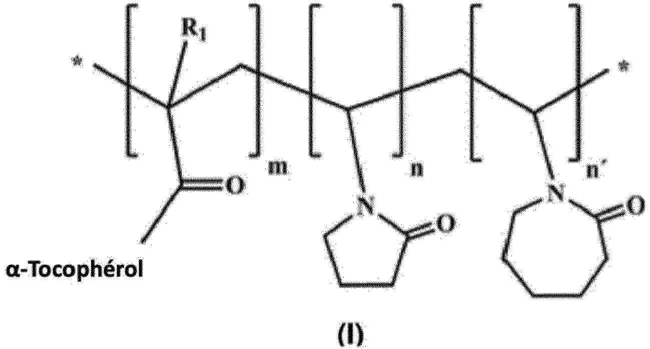
dans lequel R₁ est choisi entre l'hydrogène et un alkyle en C₁-C₄ linéaire ou ramifié ; m, n et n' représentent la fraction molaire de chacune des unités délimitées par les symboles [ ] dans la composition finale du composé polymère, dans lequel (m + n + n') = 1 et m, n et n' sont différents de zéro,
et * s'entend comme la répétition consécutive desdites unités.

2. Terpolymère de formule (II) : 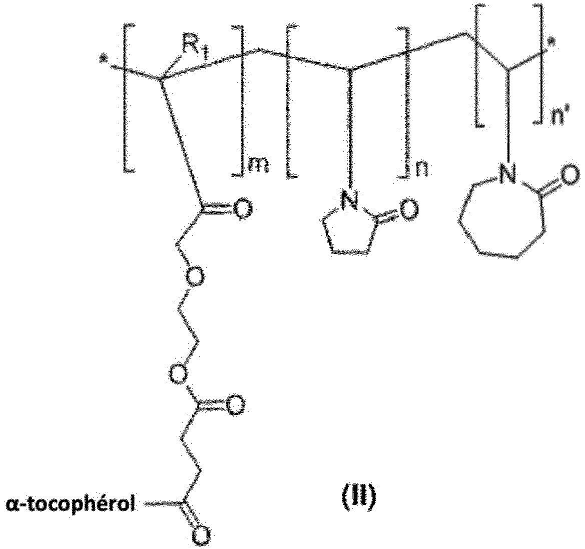 dans lequel R₁, m, n et n' sont tels que définis dans la revendication 1.

3. Terpolymère selon l'une quelconque des revendications précédentes, dans lequel m vaut 0,15.

4. Nanoparticule multi-micellaire comprenant le terpolymère selon l'une quelconque des revendications 1 à 3.

5. Nanoparticule multi-micellaire selon la revendication 4, dans laquelle ledit terpolymère est le composé de formule (I).

6. Nanoparticule multi-micellaire selon la revendication 4, dans laquelle ledit terpolymère est le composé de formule (II).

7. Nanoparticule multi-micellaire selon la revendication 4, comprenant en outre un composé bioactif.

8. Nanoparticule multi-micellaire selon la revendication 7, dans laquelle le composé bioactif est choisi dans le groupe suivant : la méthylprednisolone, la dexaméthasone, la cortisone, l'ibuprofène, le naproxène, le flurbiprofène, le kétoprofène, la vitamine C, les polyphénols, l'acide carnosique, l'astaxanthine, la vitamine B1, la vitamine B6, la vitamine B12, les dérivés de β-carotène, la lutéine, l'allantoïne, la vitamine A, l'acide folique, la vancomycine, la rifampicine, le linézolide, les sels d'ammonium quaternaire, la chlorhexidine.

9. Système de composés bioactifs à libération contrôlée comprenant la nanoparticule multi-micellaire selon l'une quelconque des revendications 4 à 8.

10. Composition pharmaceutique comprenant le système à libération contrôlée selon la revendication 9.
